# EUROPEAN PATENT APPLICATION

(11) **EP 4 179 971 A1**
(43) Date of publication of application: **17.05.2023**
(21) Application number: 22206453.7
(22) Date of filing: 09.11.2022
(51) Int. Cl.: A61B 5/11, A61B 5/00, A61H 5/00, A61B 5/18

(54) **METHOD FOR CONTINUOUS MONITORING OF EYELID DYNAMICS FOR THE PREVENTION AND TREATMENT OF DRY EYE**

(30) Priority: 10.11.2021 IT 202100028547
(71) Applicant: Aiello, Francesco, Montalbano Ionico (IT)
(72) Inventor: Aiello, Francesco, Montalbano Ionico (IT)
(74) Representative: Valente Cioncoloni, Andrea

(57) **Abstract**

Method for continuous monitoring of eyelid dynamics adapted to use components already present within the most common electronic devices to monitor and signal each time interval in which the video operator reduces their eyelid blinks; in order to reduce the dry eye of the operator, the present method is further adapted to suggest a forced blink.

## Description

### Technical field

The invention relates to an innovative method for monitoring the eyelid movements of any operator destined to spend most of their time in front of digital screens. The present invention further invites the operator to perform forced blinks if the eyelid activity proves insufficient.

### Prior art

Dry eye is a chronic disease of the eye, characterised by an altered quality/quantity of what is known as "tear film". Such an alteration is responsible for symptoms such as: dry eye, foreign body sensation, blurred vision, eye redness and pain, of varying intensity.

Since reduced eyelid blinking activity (spontaneous or forced opening and closing of the eyelids) represents a cause for the development and progression of dry eye, it is known that the use of video screens for a medium-long time is capable of reducing the number and quality of spontaneous blinks and therefore of being responsible for symptoms attributable to dry eye. Recently, several applications have been introduced to induce the video-operator to perform rest periods from the use of the video terminal in order to reduce the risk of developing eye dryness. Such applications are provided with different tools such as: assessment of the work duration, notifications on the screen to remind the user to blink voluntarily, blurring the screen to induce the worker to pause from using the video terminal.

However, none of these applications was meant to measure the number of blinks in units of time (blink count, number of blinks in one minute) and the quality of the blinks (effective blink count; partial blink count, complete or partial closing of the eyelids and duration of the complete closing) during the work session. Said parameters are, according to the prior art, the values to be taken into consideration to prevent or reduce alterations from dry eye. Given that some car manufacturers use miniature camera systems to detect the number of driver blinks in a unit of time and, in the specific case, if the number of blinks increases significantly, the system detects a probability of fatigue/drowsiness of the driver indicating a possible "microsleep".

Patent WO2020231279, issued on 19/11/2020, involves the use of a particular system integrated within eyeglasses to monitor eye activity.

To date, however, there are no systems or methods which allow to monitor spontaneous blinks without the use of external equipment, which is expensive and so far not widespread.
The object of the patent is therefore to create a method for continuous monitoring of eyelid dynamics which can control the operator forced to spend a lot of time in front of digital screens, incentivising him to increase the frequency of blinks if insufficient eyelid activity is detected.

The method object of the invention is innovative in that it does not involve the use of any external device, the steps which determine the operation of the method are performed exclusively by hardware components already present within the devices commonly used by video-operators.

### Description of the invention

According to the present invention, a method is created for continuous monitoring of eyelid dynamics which effectively solves the aforementioned problems.

To solve the problem of eye dryness, present mainly in those who must work for many hours a day in front of digital screens, it is necessary to create a blink monitoring system.

This system is not sufficient to solve the problem if there is also no signalling system, which can encourage the operator to perform more blinks or forced blinks.

In the present method object of the invention, a method is claimed which, in an embodiment thereof, can be performed by an application in a completely automatic manner. Such a method is adapted to monitor the eyelid dynamics, further calculating the time intervals which pass between spontaneous blinks.

A revolutionary element is given by the manner in which said method is performed, exploiting only hardware components already existing in the most common electronic devices.

A webcam is sufficient for monitoring the operator during working hours and a CPU is adapted to calculate and evaluate the blinking times and in particular the time between two blinks as well as the complete or incomplete closing of the eyelids during a blink.

A common timer notes the time intervals so as to issue a plurality of statistics, useful for identifying the time slots and habits which lead to the problem of dry eye for video-operators.

The continuous monitoring method comprises a plurality of steps which allow the correct execution thereof.

Switching on the computer or electronic device with which the operator will have to work for an extended period of time is obviously the first step for performing the method object of the invention.

Subsequently, the method for continuous monitoring of eyelid movements is activated, following acceptance of the privacy policy. Said step, in an embodiment thereof, is completely automatic if the present method of the invention is managed by an application previously installed on the device in use. The user may also decide to inactivate the eyelid monitoring system.

The continuous monitoring of the operator's spontaneous blinks occurs through a webcam, commonly integrated in the most common electronic devices but, in an embodiment thereof, said webcam can also not belong to the device used by the operator; an external webcam can be connected by another device while performing the same function.

The measurement of the operator's voluntary blinking intervals occurs using a timer integrated inside the electronic device in use by the operator.

Following the measurement of the intervals between blinks, the results obtained by a CPU are calculated.

The data obtained are then stored for processing a plurality of statistics related to the individual operator or for all users of the method in question, sharing the information collected and outlining an effective strategy for reducing the problem of eye dryness.

By way of non-limiting example, the storage of the obtained data provides a statistics service which is viewable by the operator at the end of the month or when the operator requests it through a plurality of graphs which illustrate the eyelid activity according to the time and according to the selected settings of the monitoring method.

The sending of a push notification occurs directly on the screen of the electronic device used by the operator; said notification is adapted to induce the operator to perform a plurality of forced blinks, if the spontaneous blinks have been insufficient or incomplete according to the CPU and the statistics previously collected.

In an embodiment thereof, said push notification is viewed by the operator by means of a message indicating the last measured eyelid activity, accompanied by a blue warning notification visible on the screen which the operator is observing. Thereby, the operator becomes aware of the problem and how insufficient his last eyelid activity was.

Further settings are customisable and, in an embodiment thereof, modular. For example, the colorimetric pattern of the screen can vary depending on the working time, in order to reduce eye dryness due to the continuous viewing of screens which are too bright with colours which are too invasive for the surrounding environment.

A further setting is that relating to the establishment of the blurring effect to obscure the screen image, in order to encourage the operator to perform multiple blinks, further trying to reduce the screen viewing time by the video-operator.

The advantages offered by the present invention are apparent in the light of the description set forth herein and will be further clarified by the accompanying figures and the detailed description.

### Description of the figures

The invention will be described below in at least one preferred embodiment by way of non-limiting example and with the aid of the accompanying figure, in which:

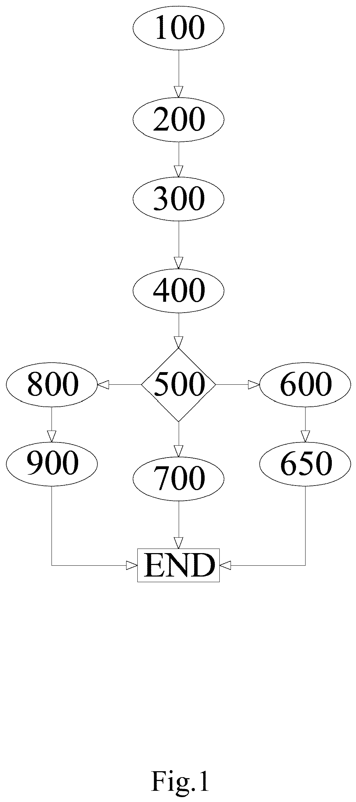

-FIGURE 1 shows the flow chart underlying the method object of the invention, connected to the monitoring method of eyelid activity which causes, if reduced, eye dryness in most video-operators.

### Detailed description of the invention

The present invention will now be illustrated by way of non-limiting or binding example, referring to the figure which illustrates some embodiments in relation to the present inventive concept.

With reference to FIG.1, the flow chart adapted to explain the plurality of steps which ensure the correct operation of the present method for continuous monitoring of eyelid movements is illustrated.

Scrolling table 1 from top to bottom and following the order of the arrows, it is possible to follow the logical order of the following steps:
- switching on 100 the computer or electronic device with which the operator will have to work for an extended period of time;
- automatic or operator-induced activation 200 (depending on the settings chosen by the operator) of the method
   for continuous monitoring of eyelid movements, by means of acceptance of the privacy policy;
- continuous monitoring 300 of the operator's spontaneous blinks through a webcam, commonly integrated in the most common electronic devices;
   - measuring 400 the operator's voluntary blinking intervals by using a timer integrated inside the electronic device in use;
   - calculation 500 of the results obtained by continuous monitoring by a CPU;
- storing 600 the data obtained for processing a plurality of statistics related to the individual operator or for all users of the method in question;
   - sending a push notification 700, displayed directly on the screen of the electronic device used by the operator, adapted to induce the operator
      to perform a plurality of forced blinks, if the spontaneous blinks have been insufficient
      or incomplete according to the CPU and the statistics previously collected;
   - variation of the colorimetric pattern 800 of the screen in relation to the working time, in order to reduce eye dryness due to the
      continuous display of overly bright screens with colours which are too invasive for the
      surrounding environment;
   - establishing the blurring effect 900 to blur the screen image, in order to encourage the operator to perform multiple blinks, further trying to reduce the screen viewing time by the video-operator.

Lastly, it is clear that the invention described up to now can be subjected to modifications, additions or variants obvious to those skilled in the art, without departing from the scope of protection outlined by the attached claims.

## Claims

1. Method for continuous monitoring of eyelid dynamics **characterised in that** it uses components already contained within the most common electronic devices, to monitor and report each time frame in which the video-operator reduces his eyelid blinks, preventing and/or treating dry eye of the video-operator; said method being further adapted to suggest forced blinking; said method comprising the steps of:
- switching on (100) the computer or the electronic device with which the operator is to work for an extended period of time;
- activation (200) automatic or operator-induced activation of the method of continuous monitoring of eyelid blinks, by acceptance of the privacy policy;
- continuous monitoring (300) of spontaneous blinks by the operator by means of a webcam, commonly integrated in the most popular electronic devices;
- measuring (400) the operator's voluntary blink ranges using a timer integrated into the electronic device in use;
- calculation (500) of the results obtained by continuous monitoring by a CPU;
- storage (600) of the data obtained for processing a plurality of statistics relating to the individual operator, subsequently available to all users of the method concerned;
- sending of a push notification (700), displayed directly on the screen of the electronic device used by the operator, suitable to induce the operator to increase the number of blinks in the unit of time, in case the spontaneous blinks were insufficient or incomplete according to the CPU and the statistics previously collected;
- variation of the colorimetric pattern (800) of the screen in relation to the working time, in order to reduce the dryness of the eye due to the continuous display of too bright screens with colours that are too invasive for the surrounding environment;
- establishment of a blurring effect (900) to blur the screen image, in order to induce the operator to perform multiple blinks, further trying to reduce the screen viewing time by the video screen operator.

2. Method for continuous monitoring of eyelid dynamics according to the preceding claim 1, **characterised in that** said webcam, dedicated to continuous monitoring (300) of spontaneous blinks by the operator, is connected with the main electronic device used by the operator while belonging to a second external device.

3. Method for continuous monitoring of eyelid dynamics, according to any one of the preceding claims, **characterised in that** said push notification is displayed by the operator by means of a message showing the last measured eyelid activity, together with blue signal notification visible on the screen that the operator is observing.

4. Method for continuous monitoring of eyelid dynamics according to any one of the preceding claims, **characterised in that** the present monitoring method has a plurality of modulable settings allowing certain controls and alerts to be kept active, customising said method at the operator's discretion.

5. Method for continuous monitoring of eyelid dynamics according to any one of the preceding claims, **characterised in that** the storage (600) of the data obtained, provides a statistics service (650) which is viewable by the operator at the end of the month or when the operator requests it by means of a plurality of graphs illustrating eyelid activity depending on the time of day and according to the monitoring method settings chosen.

6. Method for continuous monitoring of eyelid dynamics according to any one of the preceding claims, **characterised in that** the activation (200) of the continuous monitoring method is fully automatic if the present method is managed by an application previously installed on the device in use by the operator.

7. Method for continuous monitoring of eyelid dynamics according to any one of the preceding claims, **characterised in that** said timer is further suitable for measuring work intervals by means of said eyelid monitoring (300), reminding the operator to take pause periods after certain preset time ranges.
